(19) Europäisches Patentamt
European Patent Office
Office européen des brevets

(11) Publication number: **0 219 054 B1**

(12) **EUROPEAN PATENT SPECIFICATION**

(45) Date of publication of patent specification: **08.04.92**

(51) Int. Cl.[5]: **A61K 47/04**, A61K 9/06, A61K 7/40, A61K 9/12

(21) Application number: **86113910.3**

(22) Date of filing: **07.10.86**

The file contains technical information submitted after the application was filed and not included in this specification

# (54) An allergen absorbent and blocking gel.

(30) Priority: **07.10.85 US 785167**

(43) Date of publication of application:
**22.04.87 Bulletin 87/17**

(45) Publication of the grant of the patent:
**08.04.92 Bulletin 92/15**

(84) Designated Contracting States:
**AT BE CH DE ES FR GB GR IT LI LU NL SE**

(56) References cited:
**FR-A- 2 409 081**
**US-A- 2 684 321**
**US-A- 2 857 311**
**US-A- 3 922 342**

(73) Proprietor: **United Catalysts Incorporated**
**1227 South 12th Street**
**Louisville Kentucky 40232(US)**

(72) Inventor: **Powell, Thomas**
**4011 Small Boat Court**
**Louisville Kentucky 40229(US)**
Inventor: **Schulz, Anthony**
**Route 2, Box 10H**
**Floyds Knobs Indiana 47119(US)**
Inventor: **Beall, Gary W.**
**P.O. Box 1**
**Fairfield Kentucky 40020(US)**

(74) Representative: **Reitzner, Bruno, Dr.**
**Patentanwälte Dipl.-Ing. R. Splanemann Dr.**
**B. Reitzner Tal 13**
**W-8000 München 2(DE)**

EP 0 219 054 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to an allergen barrier composition for topical application to the skin to prevent the allergic reaction of persons coming into contact with poison ivy, poison oak, poison sumak or other allergens.

Poison ivy and poison oak are two of the major causes of allergic contact dermatitis in the United States today. According to Dr William Epstein, as reported in the Smithsonian, Volume 16, Number 5, dated August, 1985 by Noel Vietmeyer:

"Poison ivy and poison oak are by far the major causes of allergic contact dermatitis in the United States. More people suffer from them than from all the other allergic skin diseases combined.... No one counts the number of cases, but there are probably at least ten million a year, nationwide. Poison oak and poison ivy are possibly the greatest cause to workmen's disability in the nation; each year may bring more than 140,000 cases in the workplace, causing perhaps more than 152,000 lost work days.

The allergen common to poison oak, poison ivy and poison sumac is a heavy oil called urushiol. It is a clear, gummy, oily substance that is so incredibly reactive that only a pin-head amount can cause rashes in 500 sensitive people. Additionally, the effect of the allergen is so long lasting that botanists have received serious dermatitis from plant samples stored away for more than a century. In one reported incident, lacquer from a Chinese jar, which had been buried for almost a thousand years, resulted in dermatitis. Additionally. it does not effect animals and household pets. Cats and dogs can be exposed and actually play in the area, without being affected but can infect their owners by brushing up against their skin and transferring the urushiol on their coats to the unexposed areas of the human anatomy. According to Dr. Epstein, ibid.:

"Between 15 and 25% of us are essentially immune, 25% are mildly sensitive and don't normally develop severe reactions, 25 to 30% are moderately sensitive and break out significantly with the amount of urushiol found in one leaf and 10 to 20% are so exquisitely sensitive that less than one leaf produces intense dermatitis..."

The oily substance urushiol, when in contact with the skin, penetrates the outer skin layers and begins to chemically bind to the skin cells. The body sees the combination of the urushiol in chemical combination with a skin cell as a foreign intruder. The immune system immediately rushes large white cells called macrophages and T-lymphocytes to destroy the affected skin cell. Dr. Epstein explains, ibid.:

"It's the body's own over-reaction that causes the complications. In sensitized persons, the area fills up with the white blood cells and they release so much cell-destroying toxins that they tear apart even the skin itself. That's what produces the blisters and suppurating sores."

Many folk remedies have been proposed for use after the contact with the urushiol. These include bathing in horse urine or bleach, cleaning the skin with gasoline, morphine, gun powder, buttermilk, marshmallows or strychnine; drinking photographer's hypo; or rubbing on ammonia, mustard, Lysol®, hairspray, clear nail polish, meat tenderizer or toothpaste.

Drinking milk from goats that have grazed on poison ivy or poison oak has long been claimed to give immunity. According to Dr. Byers of the University of California's Dermatology Department (San Francisco), ibid.:

"This is one folk remedy that probably works, although it has never been tested. There remains something magic about oral doses of urushiol oil, and goat's milk probably has traces of it. If you drink goat's milk, you're probably doing about the same thing we are in our vaccine work."

The sores can be treated by physicians with various cortisone salves. However, it is essential that the salve be used for the term of the allergic reaction. The rash immediately disappears upon application of the cortisone-containing salve, but will rebound or reappear if the treatment is stopped prior to the end of the reaction.

A major problem as to the contact with urushiol from poison oak, poison sumac and poison ivy is encountered by the foresters of the U. S. Forestry Service. This is particularly severe in the case of forest fires, where the soot and gases from the burning flames contain urushiol, which can get onto the foresters fighting the fire and even into their respiratory system, causing severe complications by swelling and closing off of the esophagus and respiratory systems.

Work is under way at the University of Oregon and at the University of California as to developing tests to determine susceptibility to poison ivy and the other toxic plants containing urushiol. Other work is concerned with developing topical ointments, salves and other prophylactic creams or gels for the prevention of the allergic reaction through blocking of the urushiol oil from contact with the skin. Hundreds of materials have been tested with varying results. FR-A-2 409 081 discloses compositions containing an organophilic clay and an organic vehicle. It also discloses the use of an intermediate pre-formed gel

containing the organophilic clay in the preparation of useful aerosol compositions. The amount of organophilic clay in this pre-formed gel may range from 5 to 15 % by weight of the gel. However, in the finally prepared aerosol compositions, the amount of organophilic clay is much lower, typically 0.25 % by weight.

US-A-2 684 321 discloses a skin treating ion exchange mixture which is said to overcome venenation as from poision ivy, poison oak, poison sumac and other plants which cause dermatitis. This mixture contains organic ion exchangers, a quaternary ammonium anion exchange resin in a basic form, a carboxylic cation exchange resin in its hydrogen form, and an amino anion exchange resin in its basic form.

The aerosol allergen barrier composition for topical application according to the invention comprising:

A. a barrier composition consisting essentially of

1) from 5 to 15 % by weight of a smectite clay having a cation exchange capacity of at least 50 milliequivalents per 100 g, said clay having been ion exchanged with at least 50 milliequivalents per 100 g of said clay of a quaternary ammonium compound having at least one alkyl group containing more than 10 carbon atoms and

2) from 95 to 85 % by weight of a vehicle; and

B. an aerosol propellant.

This barrier composition, when topically applied to the skin, effectively blocks the skin from contact with urushiol and other allergens and absorbs the allergen and holds it away from the skin until it is washed off with soap and water.

Preferred embodiments of the aerosol allergen barrier composition according to the invention are recited in the dependent claims.

The quaternary ammonium compound has at least one alkyl group containing from about 10 to 22 carbon atoms.

It is essential that the organophilic clay and quaternary ammonium compound be highly activated and this is accomplished by high-shear mixing with a colloid mill or other known mechanisms and the use of a low molecular weight polar activator, such as methanol, or a self-activative material. The highly-activated gel, consisting of platelets of the smectite clay, forms a barrier on the skin, possibly through contact of the active tallow tails of the organic material with the lipids of the skin and by absorption of the urushiol in the organic tallow tails. It is felt, therefore, that there is a partitioning effect which effectively blocks and absorbs the oil phase urushiol from aqueous, phase perspiration, allowing the urushiol to be held away from the skin and held in chemical combination with the reactive tallow tails of the organo-treated clay gel, while allowing the aqueous phase materials to pass through the clay barrier.

A similar mechanism will take place with other allergens.

The invention also relates to the use of the aerosol allergen barrier composition as defined above, and of the barrier composition A as defined above, for the manufacture of a medicament for protecting the skin from contact with an allergen.

Furthermore, the invention relates to a method of preventing contamination of clothes and utensils with an allergen comprising component A as defined above.

The invention is illustrated by the attached drawings, wherein:

Fig. 1 is a diagrammatic view of the skin's surface and the organophilic clay platelets acting as a barrier to the invading urushiol droplets.

Fig. 2 is a diagrammatic view of an individual clay platelet with the alkyl groups attached thereto.

It his been known for a long time that organic compounds which contain a cation will react under favorable conditions by ion exchange with clays which contain a negative layer-lattice and exchangeable cations to form organophilic organic-clay products. If the organic cation contains at least one alkyl containing at least 10 carbon atoms, then such organo-clays have the property of swelling in certain organic liquids. See, for example, U. S-A-2,531,427 and U. S.-A-2,966,506. See also the book Clay Mineralogy, Second Edition, 1968, by Ralph E. Grim (McGraw-Hill Book Co., Inc.), particularly Chapter 10, "Clay-Mineral-Organic Reactions," pp. 356-368 - "Ionic Reactions, Smectite;" and pp. 392-401 - "Organophilic Clay-Mineral Complexes." Since the commercial introduction of these organo-clays in the early '50's, it has become well-known to gain maximum gelling (thickening) by adding a low molecular weight polar organic compound to the composition. Such polar organic compounds have been variously called activators, dispersion aids and solvating agents. See, for example, U. S-A-2,677,661; 2,979,229; 2,833,720; 2,979,229; and 3,294,683. The most efficient and accepted polar materials for use as activators have been found to be low molecular weight alcohols and ketones, particularly methanol and acetone. The activators, however, have very low flash points and require the use of flame-proof apparatus. Higher-boiling activators, having higher flash points, such as propylene carbonate, may also be used. Clays used to prepare the allergen absorbent and blocking gels of this invention are the smectite-type clays, having a high cation exchange

capacity. The cation exchange capacity of the smectite clay should equal or exceed 50 milliequivalents per 100 grams of clay. Particularly desirable types of clay are the naturally-occurring Wyoming variety of swelling bentonite and like clays as well as hectorite. a swelling magnesium-lithium silicate clay. Suitable bentonite clays are also found in Europe, particularly in the Moosburg section of Bavaria, near Munich Smectite clays can also be prepared synthetically by either a pneumatolytic or preferably a hydrothermal synthesis process. Representative hydrothermal processes for preparing synthetic smectities are disclosed in U. S-A-3,252,757 3,586,478, 3,666,407, 3,671,190, 3,844,978, 3,844,979 3,852,405 and 3,855,147.

As has been previously indicated, the invention relates to the discovery that organo-treated clays of the smectite type, which are highly activated, produce allergen absorbents and blocking gels for topical application to the skin to prevent contact of the skin with the allergens produced by poison ivy, poison oak or poison sumac or with other allergens. The smectite-type clays, which have been sufficient cation exchange capacity to be ion exchanged with organic compounds having a cation and one or more alkyl chains, having at least 10 carbon atoms naturally occur in Wyoming and in the Moosburg section of Bavaria, in the vicinity of Munich, Germany. The clays are of the bentonite type and are usually of the sodium form. However, if they are not already in the sodium form, they can be converted by passing an aqueous clay slurry through a bed of cation exchange resin in the sodium form. Alternately, the smectite clay can be mixed with water and a soluble sodium compound, such as sodium carbonate, sodium hydroxide and sheared at high shear in a pug mill or extruder. Additionally, smectite-type clays can be prepared synthetically, either by a pneumatolytic, or preferably a hydrothermal, synthesis process. Representatives of such smectite clays are the following:

Montmorillonite

$[(Al_{4-x}Mg_x)\ Si_8O_{20}(OH)_{4-f}F_f]x\ R^+$ where $0.55 \leqq x \leqq 1.10$, $f \leqq 4$ and R is selected from Na, Li, $NH_4$, and mixtures thereof;

Bentonite

$[(Al_{4-x}Mg_x(Si_{8-y}Al_y)O_{20}(OH_{4-f}F_f](x+y)\ R^+$ where $0 < x < 1.10$, $0 < y < 1.10$, $0.55 \leqq (x+y) \leqq 1.10$, $f \leqq 4$ and R is selected from Na, Li, $NH_4$, and mixtures thereof;

Beidellite

$[(Al_{4-y})\ (Si_{8-y}Al_{x+y})O_{20}(OH)_{4-f}F_f]x\ R^+$ where $0.55 \leqq x \leqq 1.10$, $0 \leqq y \leqq 0.44$, $f \leqq 4$ and R is selected from Na, Li, $NH_4$, and mixtures thereof;

Hectorite

$[(Mg_{6-x}Li_x)Si_8O_{20}(OH)_{4-f}F_f]x\ R^+$ where $0.57 \leqq x \leqq 1.15$, $f \leqq 4$ and is selected from Na, Li, $NH_4$, and mixtures thereof;

Saponite

$[(Mg_{6-y}Al_y)\ (Si_{8-x-6}Al_{x+y})O_{20}(OH)_{4-f}F_f]x\ R^+$ where $0.58 \leqq x \leqq 1.18$, $0 \leqq y \leqq 0.66$, $f \leqq 4$ and $R^+$ is selected from Na, Li, $NH_4$, and mixtures thereof;

Stevensite

$[(Mg_{6-x}Al_y)Si_8O_{20}(OH)_{4-f}F_f]2\ x\ R^+$ where $0.28 \leqq x \leqq 0.57$, $f \leqq 4$ and R is selected from Na, Li, $NH_4$, and mixtures thereof.

These smectite clays may be synthesized hydrothermally by forming an aqueous reaction mixture in the form of a slurry containing mixed hydrous oxides or hydroxides of the desired metals with or without, as the case may be, sodium (or alternate exchangeable cation or mixture thereof) fluoride in the proportions defined in the above formulas and preselected values of x, y and f for the particular synthetic smectite desired. The slurry is then placed in an autoclave and heated under autogenous pressure to a temperature within the range of approximately 100° to 325° C, preferably 275° to 300° C for a sufficient period of time to form the desired product. Formulation times of 3 to 48 hours are typical at 300° C, depending on the particular smectite being synthesized and the optimum time an be readily determined by pilot trials. The

organic compounds useful in this invention are quaternary ammonium salts containing one methyl radical or one benzyl radical and a mixture of trialkyl radicals, having from 14 to 20 carbon atoms, preferably wherein 20 to 35% have 16 carbon atoms and 5% have 18 carbon atoms on a 100% basis. The anion salt is preferably selected from chloride and bromide and mixtures thereof and is preferably a chloride. However, other anions, such as acetate, hydroxide, nitrite, etc., may be present in the ammonium salt to neutralize the quaternary ammonium compound. The methyl or benzyl trialkyl ammonium salt may be represented as follows:

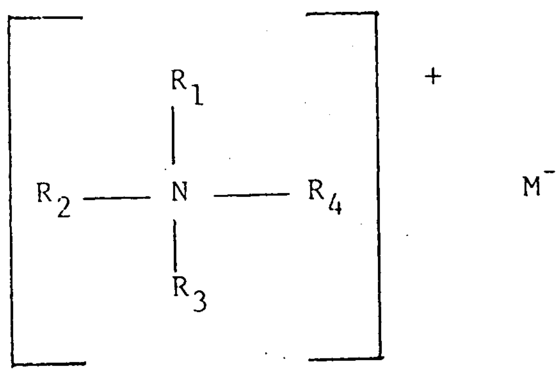

The preferred quaternary amine for use in the practice of this invention is dimethyl dihydrogenated tallow ammonium chloride. $R_1$ can be methyl or benzyl, $R_2$ can be methyl or $C_{10}$ to $C_{18}$. $R_3$ can be methyl or $C_{10}$ to $C_{18}$. $R_4$ can be $C_{10}$ to $C_{18}$. Commercially-prepared hydrogenated tallow typically analyzes 2.0% $C_{14}$, 0.5% $C_{15}$, 29.0% $C_{16}$, 1.5% $C_{17}$, 66.0% $C_{18}$ and 1.0% $C_{20}$ alkyl radicals.

Nevertheless, the alkyl radicals may be derived from other natural oils, including various vegetable oils, such as corn oil, soybean oil, cottonseed oil, or castor oil, as well as various animal oils and fats. Additionally, the alkyl radicals may be petrochemically derived, as from alpha olefins.

We have found that it is essential that the organophilic clay compounds be highly activated. Alternately, self-activating organophilic clays may be used which do not require activation with polar materials. Activation of the organophilic clays may be accomplished by use of organic polar materials of low molecular weight (which are known as activators). The most efficient and accepted polar materials are low molecular weight alcohols and ketones, particularly methanol and acetone. However, these activators do have the disadvantage of a very low flash point and therefore the danger of combustion is present. Higher boiling flash point activators, such as propylene carbonate, therefore, may be advantageously utilized. The organophilic clays are prepared by admixing the smectite clay, the quaternary ammonium compound and water together, preferably at temperatures within the range of 100° to 180°F (38° to 77°C) for a period of time sufficient for the organic compound to coat the smectite clay particle, followed by filtering, washing, drying and grinding. In using the organophilic clays in emulsions, the drying and grinding steps can be eliminated. When the smectite clay and quaternary ammonium compound and water are mixed together in such concentrations that a slurry is not formed, then the filtration and washing steps are eliminated. Preferably, however, the smectite clay is dispersed in water at a concentration of from about 3 to 7% and the slurry is optionally centrifuged to remove nonclay impurities. These nonclay impurities may constitute between about 10 to 50% of the starting clay composition. Thereafter, the slurry is agitated and heated to a temperature in the range of 104° to 170°F (60° to 77°C), the quaternary amine salt added in the proper milliequivalent ratio, preferably as a liquid in isopropanol. The amount of the quaternary ammonium salt added to the smectite clay for purposes of this invention must be sufficient to impart to the organophilic clay the enhanced dispersion characteristics desired. Milliequivalent ratio is defined as the number of milliequivalents of the organic compound in the organo-clay per 100 g of clay, on a 100% active clay basis. The organophilic clays preferably have a milliequivalent ratio of from 100 to 120. However, if polar organic activators are utilized, a much lower milliequivalent ratio can be utilized without affecting the resultant gel system disadvantageously. The smectite clay and quaternary ammonium compound are admixed with a hydrocarbon vehicle or a volatile silicone vehicle and the entire admixture is subjected to a high-shear mixing in a colloid mill. Generally speaking, the concentration of the organo-treated clay is in the ratio of from 5 to 15% by weight. Therefore, the vehicle for the resulting gel makes up the balance of the mixture in the weight concentration of from about 95 to 85%.

The gel is utilized in the form of an aerospray, the admixture of organo-treated clay forming the gel

being in the ratio of 5 to 10% and the total gel making up about 30% of the contents of the can, with the rest of the material being a propellant. Typical aerosol propellants are hydrocarbon blends of isobutane. The trade designations are A46™, A31™ and A70™.

Referring now to Fig. 1, there is illustrated a diagrammatic section of the skin after a diagram of Gray's Anatomy. As will be noted, the skin is made up of an epidermis 1 and a dermis 7. The epidermis consists of five layers, the stratum corneum 2, the stratum lucideum 3, the stratum granulosum 4, the stratum mucosum 5, which terminates in the stratum germinationium 6. The sudoriferous gland 8 is located in the dermis. However, the ducts 9 extend through the epidermis to the outer layer of the skin. Additionally, the shaft of the hair 10 extends through duct 14 and terminates at the bulb 12. The hair follicle is surrounded by sebaceous glands 11, which discharge into the duct 14 to keep the hair shaft lubricated. Additionally, the dermis / contains some relatively deep-lying arteries 21 and nerves 22.

According to the drawing, the organo-treated clay 15, made up of platelets 16 and the depending tallow tails 17 form as a layer over the skin with the tallow tails reacting to some extent with the lipids of the skin's surface. This allows the platelets to align themselves pretty much as a shield against the invading urushiol droplets 18. The droplets, in order to reach the skin, must pass through the barriers or blockers formed by the platelets 16 without being absorbed by the reactive organic alkyl groups in the form of tallow tails 17. It is believed that the tallow tails, through the van-der Waal forces, tend to absorb chemically the urushiol droplets and hold the urushiol droplets and thus prevent their contact with the skin (see Fig. 2). Any urushiol droplets which might escape the first row of tallow tails are blocked by the clay platelets 16 and then encounter succeeding alkyl groups where absorption takes place.

Urushiol 18 is diagrammatically illustrated in Fig. 2, according to its chemical formula. As will be noted, the urushiol compound consists of a phenyl group 20 with two hydroxyl groups and a long hydrocarbon chain of 15 to 17 carbons, designated as 19.

Additionally, the quaternary ammonium compounds are designated with the tallow tails 17, consisting of 16 to 18 carbons. Some of the quaternary ammonium compounds contain a benzyl compound and one alkyl chain and two methyl groups, while the other quaternary ammonium compounds consist of two alkyl hydrocarbon chains 17 and two methyl groups. The van-der Waal bonding of the hydrocarbon chain 19 of the urushiol compound 18 is diagrammatically shown with the long-chain alkyl group 17 of the organophilic clay 15. Additionally, it is felt that the phenyl group 20 of the urushiol compound may have some affinity for the active surface of the platelet 16 of the organophilic clay. This is shown diagrammatically in the right-hand portion of Fig. 2.

It should be understood that this explanation is somewhat hypothetical. However, it is supported by data, as will hereinafter be shown. The fact of the matter is that adsorption apparently is not the major mechanism in this instance, since materials with higher surface areas do not provide the protection that is provided by the organo-treated clays. It is felt, therefore, that the organic surface area is of major importance while the inorganic surface area formed by the pores of the clay platelets perform only a secondary function relative to the absorptive function performed by the reactive tallow tails of the organophilic clays.

The invention will be better understood by reference to the examples contained hereinafter.

EXAMPLE 1

A batch of organophilic clay is prepared by admixing finely-milled western sodium bentonite with a quaternary ammonium compound obtained from hydrogenated tallow. This quaternary ammonium compound contains two alkyl groups from 16 to 18 carbons in length and two methyl groups. The quaternary ammonium compound is admixed with water and the sodium bentonite at a temperature within the range of 100° to 180°F(38 to 82°C) for a period of time sufficient for the quaternary ammonium compound to coat the clay particles. The sodium bentonite then reacts with the reactive chlorine or other anion, so that the quaternary ammonium compound becomes ion exchanged with the bentonite clay particles. A polar organic activator, as for example propylene carbonate, may he added to the admixture, which is then mixed with a long-chain hydrocarbon vehicle or a volatile silicon liquid and subjected to high shearing in a colloid mill.

EXAMPLE 2

The test procedure* involved the application of Sure® deodorant and the organophilic clay of Example 1, spray applied to the skin of the forearm at 1,4 and 24 hours before the application of dilutions of purified urushiol in acetone in a double-blind fashion. Three samples of urushiol in acetone were made up with five microliters of urushiol in acetone, ranging in amount from .25ug to .005ug. The samples of urushiol in acetone were then applied to the patch test sites. The patch test sites were there after read two to five days later and recorded from N to 4 as follows:

N = Normal
1 = Erythema and edema involving half the test site
2 = Erythema and edema plus small vesicles involving the entire site
3 = Full involvement of the test site with erythema and edema and large vesicles
4 = Bullae

A -1 score equals a definite positive reaction which invoves less than 1/2 of the test site and a ± equals a questionable rejection which either is subsequently read as, ± or -.

EXAMPLE 3

A second preliminary study was carried out, comparing the results of pretreatment with Sure® to pretreatment with Drysol® (a 20% w/v concentrate of aluminum chloride hexahydrate in alcohol). The pretreatment time was 4 hours. The patch tests were urushiol and the patch test readings were the same as described above.

EXAMPLE 4

In the next series of experiments, the subjects were pretreated with breakdown products of Sure® that either were missing the aluminum chloralhydrate or the suspending agents (hectorite and propylene carbonate). The patch tests with urushiol and the patch test readings were the same as described above.

The results of these tests are shown in Table I.

TABLE I

Average Protective Effect of SURE® and Example 1 Compared to the Control

| Pretreatment Time | Microgram Dilutions of Urushiol | | | |
|---|---|---|---|---|
| 1 hour | 0.25 | 0.1 | 0.05 | 0.025 |
| Sure® | 1 | 1.3 | 1.2 | 1.0 |
| Example 1 | Not Done | | | |
| 4 hours | | | | |
| Sure® | 1.5 | 0.9 | 0.9 | 0.5 |
| Example 1 | 3.0 | 2.0 | 1.8 | 1.3 |
| 24 hours | | | | |
| Sure® | 0 | 0.75 | 1.0 | 0.4 |
| Example 1 | 1.5 | 0.9 | 0.9 | 1.5 |

Table I shows that pretreatment with the Sure® deodorant does reduce the patch test reaction to dilutions of urushiol. However, Sure® is not able to completely prevent the reactions, but simply reduce them. The material of Example 1, on the other hand, was more effective at reducing the reactions and this was particularly noticeable at 4 and 24 hours after applications. as compared to Sure®. This table averages

*These tests were conducted under a grant from the U.S. Forestry Service under the supervision of Dr. William Epstein of the University of California Medical School at San Francisco.

overall responses.

The preliminary study, comparing the high concentration of the aluminum salt (Drysol®) to Sure®, indicated that the alcoholic solution was less effective than Sure®.

Another experiment compared the blocking effect of Sure® with its ingredients, i.e. without fillers and without aluminum chloralhydrate. In one instance, Sure® was compared to the aluminum compound containing preparation without the fillers, i.e. the organophilic hectorite, and the two were equal on two occasions. Sure® was more effective in one and definitely more effective in four instances. In no instance, was the aluminum salt more effective than Sure®. Sure®, containing only the fillers and no aluminum was compared to Sure® and the two preparations were equal on two occasions. Sure® was more effective than the filler preparations on two occasions and much more effective on one occasion. On the other hand, the filler was more effective than Sure® on two occasions. Finally, in direct comparison of the filler versus the aluminum preparation, the filler was more effective than the aluminum salt on two occasions and much more effective in four additional trials. In one instance, the aluminum salt was, more effective than the filler preparations.

EXAMPLE 5

The tests, as previously described, were carried out with three particularly sensitive individuals. These are shown in Tables II, III and IV.

As can be seen, after the second day, the control showed a normal reaction for the patch test for the low concentrations, but a 2 reaction as to any concentration

TABLE III

| | | Day 2 | | | Day 4 | |
|---|---|---|---|---|---|---|
| micrograms | Urushiol applied 4 hours after application of protective composition | | | | | |
| Urushiol | Control | Sure® | Ex. 1 | Control | Sure® | Ex. 1 |
| .25 | | | | | | |
| .1 | 2 | 2 | N | 4 | 2 | N |
| .05 | 2 | 2 | N | 3 | 2 | N |
| .025 | 1 | -1 | N | 2 | 1 | N |
| .01 | -1 | N | N | N | N | N |

| | | Day 2 | | | Day 4 | |
|---|---|---|---|---|---|---|
| micrograms | Urushiol applied 24 hours after application of protective composition | | | | | |
| Urushiol | Control | Sure® | Ex. 1 | Control | Sure® | Ex. 1 |
| .25 | | | | | | |
| .1 | 2 | 2 | 2 | 4 | 2 | 2 |
| .05 | 2 | 2 | 2 | 3 | 2 | 2 |
| .025 | 1 | 1 | N | 2 | 1 | N |
| .01 | -1 | N | N | N | N | N |

TABLE III

| | | Day 2 | | | Day 4 | |
|---|---|---|---|---|---|---|
| micrograms | Urushiol applied 4 hours after application of protective composition | | | | | |
| Urushiol | Control | Sure® | Ex. 1 | Control | Sure® | Ex. 1 |
| .25 | | | | | | |
| .1 | 2 | 2 | N | 4 | 2 | N |
| .05 | 2 | 2 | N | 3 | 2 | N |
| .025 | 1 | -1 | N | 2 | 1 | N |
| .01 | -1 | N | N | N | N | N |

| | | Day 2 | | | Day 4 | |
|---|---|---|---|---|---|---|
| micrograms | Urushiol applied 24 hours after application of protective composition | | | | | |
| Urushiol | Control | Sure® | Ex. 1 | Control | Sure® | Ex. 1 |
| .25 | | | | | | |
| .1 | 2 | 2 | 2 | 4 | 2 | 2 |
| .05 | 2 | 2 | 2 | 3 | 2 | 2 |
| .025 | 1 | 1 | N | 2 | 1 | N |
| .01 | -1 | N | N | N | N | N |

TABLE IV

| micrograms | Day 2 | | | Day 4 | | |
|---|---|---|---|---|---|---|
| | Urushiol applied 4 hours after application of protective composition | | | | | |
| Urushiol | Control | Sure® | Ex. 1 | Control | Sure® | Ex. 1 |
| .05 | 2 | 2 | N | 3 | 2 | -1 |
| .025 | N | N | N | 1 | N | N |
| .01 | N | N | N | N | N | N |

| micrograms | Day 2 | | | Day 4 | | |
|---|---|---|---|---|---|---|
| | Urushiol applied 24 hours after application of protective composition | | | | | |
| Urushiol | Control | Sure® | Ex. 1 | Control | Sure® | Ex. 1 |
| .05 | 2 | 1 | 1 | 3 | 2 | 2 |
| .025 | N | -1 | N | 1 | N | N |
| .01 | N | N | N | N | N | N |

TABLE II

| | Day 2 | | | Day 4 | | |
|---|---|---|---|---|---|---|
| micrograms | Urushiol applied 4 hours after application of protective composition | | | | | |
| Urushiol | Control | Sure® | Ex. 1 | Control | Sure® | Ex. 1 |
| .25 | 2 | -1 | N | 3 | 3 | N |
| .1 | 2 | 1 | N | 2 | 1 | N |
| .05 | 2 | N | N | 2 | N | N |
| .025 | N | N | N | N | N | N |
| .01 | N | N | N | N | N | N |

| | Day 2 | | | Day 4 | | |
|---|---|---|---|---|---|---|
| micrograms | Urushiol applied 24 hours after application of protective composition | | | | | |
| Urushiol | Control | Sure® | Ex. 1 | Control | Sure® | Ex. 1 |
| .25 | 2 | 1 | N | 3 | 3 | 1 |
| .1 | 2 | 1 | N | 2 | 1 | 1 |
| .05 | 2 | N | N | 2 | N | N |
| .025 | N | N | N | N | N | N |
| .01 | N | N | N | N | N | N |

of urushiol above 0.05 µg. Sure®, on the other hand, provided some protection at 0.05 µg and reduced in the size of the reaction as to the concentrations above 0.1. The material of Example 1, however, showed full protection 4 hours after application for all concentrations of the material. Essentially the same results were obtained after Day 4. Except with the control, the severity of the reactions increased. The severity of the reaction of the high concentration of urushiol increased with the Sure® application but the material of Example 1 gave full protection throughout the total range of concentration. 24 hours after application, essentially the same results were obtained on Day 2. On Day 4, the severity of the reaction was greater with the control and with the Sure® sample and there was a 1 range of reaction for the Example 1 material after 24 hours.

Table III, for a completely different individual, who was extremely sensitive, demonstrated a more severe reaction with both the control sample and the Sure® sample, after Day 2 and Day 4, as compared to Table II. However, the organophilic clay of Example 1 provided good protection, both for Day 2 and Day 4 throughout the entire range of urushiol concentrations. The severity of the reactions increased across the board after application and even the material of Example 2 showed a moderate grade 2 reaction for the higher concentrations after the second and fourth days. The superiority of the material of Example 1 over the control and over the Sure® is shown, however, throughout.

Table IV demonstrates in like manner the protection afforded by the individual throughout a smaller range of urushiol concentrations. After the fourth day, from hours after application, the material of Example 1 showed a positive reaction, as indicated by -1. In like manner, the table demonstrates that even 24 hours

after application, there is protection against the low concentrations of urushiol by the organophilic clay of Example 1 on the second and fourth days following application.

A series of other over-the-counter preparations, Technu®, Less Ivy®, and Off®, were completely ineffective in protecting the skin from the effects of urushiol.

It is believed that the long $C_{18}$ chain of the urushiol molecule is absorbed through van-der Waal forces with the long, $C_{16}$ to $C_{22}$ carbon tallow tails of the organophilic clays. Additionally, it is felt that the phenyl group of the urushiol may have some affinity for the active surface of the clay platelet itself. The material may be applied in aerosol form, as a salve, or as a stick, onto the skin, prior to encountering the urushiol-producing plants, such as poison ivy, oak, sumac. The comparative study, however, has clearly shown that the organophilic clays of this invention are more effective than any material heretofore known in the prevention of experimentally-induced poison oak or ivy dermatitis.

## Claims

### Claims for the following Contracting States : BE, CH, DE, FR, GB, IT, LI, LU, NL, SE

**1.** An aerosol allergen barrier composition for topical application comprising:
A. a barrier composition consisting essentially of
1) from 5 to 15 % by weight of a smectite clay having a cation exchange capacity of at least 50 milliequivalents per 100 g, said clay having been ion exchanged with at least 50 milliequivalents per 100 g of said clay of a quaternary ammonium compound having at least one alkyl group containing more than 10 carbon atoms and
2) from 95 to 85 % by weight of a vehicle; and
B. an aerosol propellant.

**2.** The aerosol composition of Claim 1 comprising from 20 to 35 parts by weight of said barrier composition and 80 to 65 parts by weight of said aerosol propellant.

**3.** The aerosol composition of Claim 1 comprising 30 parts by weight of said barrier composition and 70 parts by weight of said aerosol propellant.

**4.** The aerosol composition of anyone of Claims 1 to 3, wherein said smectite clay is a highly activated clay.

**5.** The aerosol composition of anyone of Claims 1 to 4, wherein said vehicle is a long chain hydrocarbon.

**6.** The aerosol composition of anyone of Claims 1 to 4, wherein said vehicle is a silicone fluid.

**7.** The aerosol composition of anyone of Claims 1 to 6, wherein said quaternary ammonium compound contains one methyl or benzyl radical and three alkyl radicals having from 14 to 20 carbon atoms.

**8.** The aerosol composition of anyone of Claims 1 to 7, wherein said smectite clay is bentonite or hectorite.

**9.** The aerosol composition of anyone of Claims 1 to 8, additionally comprising a low molecular weight polar organic activator for said smectite clay.

**10.** The aerosol composition of Claim 9, wherein said activator is propylene carbonate or a low molecular weight alcohol or ketone.

**11.** The aerosol composition of anyone of Claims 1 to 10, wherein said propellant is a hydrocarbon blend containing isobutane.

**12.** The use of the aerosol allergen barrier composition according to anyone of Claims 1 to 11 for the manufacture of a medicament for protecting the skin from contact with an allergen.

**13.** The use of the aerosol allergen barrier composition according to part A of Claim 1 and anyone of Claims 4 to 10, for the manufacture of a medicament for protecting the skin from contact with an allergen.

**14.** A method of preventing contamination of clothes and utensils with an allergen comprising applying to said clothes and utensils a barrier composition comprising
1) from 5 to 15 % by weight of a smectite clay having a cation exchange capacity of at least 50 milliequivalents per 100 g, said clay having been ion exchanged with at least 50 milliequivalents per 100 g of said clay of a quaternary ammonium compound having at least one alkyl group containing more than 10 carbon atoms, and
2) from 95 to 85 % by weight of a vehicle.

**Claims for the following Contracting States : AT, ES, GR**

**1.** A process of preparing an aerosol allergen barrier composition for topical application comprising:
A. a barrier composition consisting essentially of
1) from 5 to 15 % by weight of a smectite clay having a cation exchange capacity of at least 50 milliequivalents per 100 g, said clay having been ion exchanged with at least 50 milliequivalents per 100 g of said clay of a quaternary ammonium compound having at least one alkyl group containing more than 10 carbon atoms and
2) from 95 to 85 % by weight of a vehicle; and
B. an aerosol propellant
which process comprises mixing component A 1 with component A 2 and adding component B to said mixture.

**2.** The process of Claim 1, wherein the aerosol composition comprises from 20 to 35 parts by weight of said barrier composition and 80 to 65 parts by weight of said aerosol propellant.

**3.** The process of Claim 1, wherein the aerosol composition comprises 30 parts by weight of said barrier composition and 70 parts by weight of said aerosol propellant.

**4.** The process of anyone of Claims 1 to 3, wherein said smectite clay is a highly activated clay.

**5.** The process of anyone of Claims 1 to 4, wherein said vehicle is a long chain hydrocarbon.

**6.** The process of anyone of Claims 1 to 4, wherein said vehicle is a silicone fluid.

**7.** The process of anyone of Claims 1 to 6, wherein said quaternary ammonium compound contains one methyl or benzyl radical and three alkyl radicals having from 14 to 20 carbon atoms.

**8.** The process of anyone of Claims 1 to 7, wherein said smectite clay is bentonite or hectorite.

**9.** The process of anyone of Claims 1 to 8, wherein the composition additionally comprises a low molecular weight polar organic activator for said smectite clay.

**10.** The process of Claim 9, wherein said activator is propylene carbonate or a low molecular weight alcohol or ketone.

**11.** The process of anyone of Claims 1 to 10, wherein said propellant is a hydrocarbon blend containing isobutane.

**12.** The use of the aerosol allergen barrier composition prepared according to anyone of Claims 1 to 11 for the manufacture of a medicament for protecting the skin from contact with an allergen.

**13.** The use of the aerosol allergen barrier composition prepared according to part A of Claim 1 and anyone of Claims 4 to 10, for the manufacture of a medicament for protecting the skin from contact with an allergen.

**14.** A method of preventing contamination of clothes and utensils with an allergen comprising applying to said clothes and utensils a barrier composition comprising
1) from 5 to 15 % by weight of a smectite clay having a cation exchange capacity of at least 50 milliequivalents per 100 g, said clay having been ion exchanged with at least 50 milliequivalents per

100 g of said clay of a quaternary ammonium compound having at least one alkyl group containing more than 10 carbon atoms, and
2) from 95 to 85 % by weight of a vehicle.

## Revendications
### Revendications pour les Etats contractants suivants : BE, CH, DE, FR, GB, IT, LI, LU, NL, SE

**1.** Composition de barrière aux allergènes, sous forme d'aérosol, en vue d'une application topique,
(A) une composition barrière consitant essentiellement en :
(1) de 5 à 15% en poids d'une argile de type smectite, ayant une capacité d'échange de cations d'au moins 50 milliéquivalents par 100 g, ladite argile ayant été soumise à un échange d'ions avec au moins 50 milliéquivalents par 100 g de ladite argile d'un composé d'ammonium quaternaire ayant au moins un groupe alkyle contenant plus de 10 atomes de carbone ; et
(2) de 95 à 85% en poids d'un véhicule ; et
(B) un propulseur pour aérosol.

**2.** Composition aérosol selon la revendication 1, comprenant de 20 à 35 parties en poids de ladite composition barrière et de 80 à 65 parties en poids dudit propulseur pour aérosol.

**3.** Composition aérosol selon la revendication comprenant 30 parties en poids de ladite composition barrière et 70 parties en poids dudit propulseur pour aérosol.

**4.** Composition aérosol selon l'une quelconque des revendications 1 à 3, dans laquelle ladite argile de type smectite est une argile hautement activée.

**5.** Composition aérosol selon l'une quelconque des revendications 1 à 4, dans laquelle ledit véhicule est un hydrocarbure à longue chaîne.

**6.** Composition aérosol selon l'une quelconque des revendications 1 à 4, dans laquelle ledit véhicule est un fluide de type silicone.

**7.** Composition aérosol selon l'une quelconque des revendications 1 à 6, dans laquelle ledit composé d'ammonium quaternaire contient un radical méthyle ou benzyle et trois radicaux alkyle ayant de 14 à 20 atomes de carbone.

**8.** Composition aérosol selon l'une quelconque des revendications 1 à 7, dans laquelle ladite argile de type smectite est la bentonite ou l'hectorite.

**9.** Composition aérosol selon l'une quelconque des revendications 1 à 8, comprenant en outre un activateur organique polaire de faible masse moléculaire pour ladite argile de type smectite.

**10.** Composition selon la revendication 9, dans laquelle ledit activateur est le carbonate de propylène ou un alcool ou une cétone de faible masse moléculaire.

**11.** Composition aérosol selon l'une quelconque des revendications 1 à 10, dans laquelle ledit propulseur est un mélange, d'hydrocarbures contenant de l'isobutane.

**12.** Utilisation de la composition de barrière aux allergènes sous forme d'aérosol, telle que définie à l'une quelconque des revendications 1 à 11, pour la fabrication d'un médicament pour protéger la peau du contact avec un allergène.

**13.** Utilisation de la composition de barrière aux allergènes sous forme d'aérosol, telle que définie à la partie (A) de la revendication 1 et à l'une quelconque des revendications 4 à 10, pour la fabrication d'un médicament pour protéger la peau du contact avec un allergène.

**14.** Procédé pour empêcher la contamination des vêtements et des ustensiles par un allergène, comprenant l'application sur lesdits vêtements et ustensiles d'un composition barrière comprenant :
(1) de 5 à 15% en poids d'une argile de type smectite, ayant une capacité d'échange de cations

d'au moins 50 milliéquivalents par 100 g, ladite argile ayant été soumise à un échange d'ions avec au moins 50 milliéquivalents par 100 g de ladite argile d'un composé d'ammonium quaternaire ayant au moins un groupe alkyle contenant plus de 10 atomes de carbone ; et
(2) de 95 à 85% en poids d'un véhicule.

**Revendications pour l'Etats contractant suivant : AT, ES, GR**

**1.** Procédé de fabrication d'une composition de barrière aux allergènes, sous forme d'aérosol, en vue d'une application topique, comprenant :
(A) une composition barrière consistant essentiellement en :
(1) de 5 à 15% en poids d'une argile de type smectite, ayant une capacité d'échange de cations d'au moins 50 milliéquivalents par 100 g, ladite argile ayant été soumise à un échange d'ions avec au moins 50 milliéquivalents par 100 g de ladite argile d'un composé d'ammonium quaternaire ayant au moins un groupe alkyle contenant plus de 10 atomes de carbone ; et
(2) de 95 à 85% en poids d'un véhicule ; et
(B) un propulseur pour aérosol,
lequel procédé compend le mélange du composent (A1) avec le composant (A2) et l'addition du composant (B) audit mélange.

**2.** Procédé selon la revendication 1, dans lequel la composition aérosol comprend de 20 à 35 parties en poids de ladite composition barrière et de 80 à 65 parties en poids dudit propulseur pour aérosol.

**3.** Procédé selon la revendication 1, dans lequel le composition aérosol comprend 30 parties en poids de ladite composition barrière et 70 parties en poids dudit propulseur pour aérosol.

**4.** Procédé selon l'une quelconque des revendications 1 à 3, dans lequel ladite argile de type smectite est une argile hautement activée.

**5.** Procédé selon l'une quelconque des revendications 1 à 4, dans lequel ledit véhicule est un hydrocarbure à longue chaîne.

**6.** Procédé selon l'une quelconque des revendications 1 à 4, dans lequel ledit véhicule est un fluide de type silicone.

**7.** Procédé selon l'une quelconque des revendications 1 à 6, dans lequel ledit composé d'ammonium quaternaire contient un radical méthyle ou benzyle et trois radicaux alkyle ayant de 14 à 20 atomes de carbone.

**8.** Procédé selon l'une quelconque des revendications 1 à 7, dans lequel ladite argile de type smectite est la bentonite ou l'hectorite.

**9.** Procédé selon l'une quelconque des revendications 1 à 8, dans lequel la composition comprend en outre un activateur organique polaire de faible masse moléculaire pour ladite argile de type smectite.

**10.** Procédé selon la revendication 9, dans lequel ledit activateur est le carbonate de propylène ou un alcool ou une cétone de faible masse moléculaire.

**11.** Procédé selon l'une quelconque des revendications 1 à 10, dans lequel ledit propulseur est un mélange d'hydrocarbures contenant de l'isobutane.

**12.** Utilisation de la composition de barrière aux allergènes sous forme d'aérosol, préparée conformément à l'une quelconque des revendications 1 à 11, pour la fabrication d'un médicament pour protéger la peau du contact avec un allergène.

**13.** Utilisation de la composition de barrière aux allergènes sous forme d'aérosol, préparée conformément à la partie (A) de la revendication 1 et à l'une quelconque des revendications, 4 à 10, pour la fabrication d'un médicament pour protéger la peau du contact avec un allergène.

14. Procédé pour empêcher la contamination des vêtements et des ustensiles par un allergène, comprenant l'application sur lesdits vêtements et ustensiles d'une composition barrière comprenant :
    (1) de 5 à 15% en poids d'une argile de type smectite, ayant une capacité d'échange de cations d'au moins 50 milliéquivalents par 100 g, ladite argile ayant été soumise à un échange d'ions avec au moins 50 milliéquivalents par 100 g, de ladite argile d'un composé d'ammonium quaternaire ayant au moins un groupe alkyle contenant plus de 10 atomes de carbone ; et
    (2) de 95 à 85% en poids d'un véhicule.

**Patentansprüche**

**Patentansprüche für folgende Vertragsstaaten : BE, CH, DE, FR, GB, IT, LI, LU, NL, SE**

1. Stoffzusammensetzung in Aerosolform zum Blockieren von Allergenen, für die lokale Anwendung, enthaltend:
    A. eine Blockier-Zusammensetzung, im wesentlichen bestehend aus
        1) 5 bis 15 Gew.% eines smektitischen Tons mit einer Kationenaustauschfähigkeit von mindestens 50 mVal je 100 g Ton, der mit einer quaternären Ammoniumverbindung mit mindestens einer Alkylgruppe, enthaltend mehr als 10 Kohlenstoffatome ionenausgetauscht worden ist, und
        2) 95 to 85 Gew.% eines Trägers; und
    B. ein Aerosol-Treibmittel.

2. Stoffzusammensetzung in Aerosolform nach Anspruch 1, enthaltend 20 bis 35 Gewichtsteile Blockierzusammensetzung und 80 bis 65 Gewichtsteile Aerosol-Treibmittel.

3. Stoffzusammensetzung in Aerosolform nach Anspruch 1, enthaltend 30 Gewichtsteile Blockierzusammensetzung und 70 Gewichtsteile Aerosol-Treibmittel.

4. Stoffzusammensetzung in Aerosolform nach einem der Ansprüche 1 bis 3, worin der smektitische Ton einen hochaktivierten Ton darstellt.

5. Stoffzusammensetzung in Aerosolform nach einem der Ansprüche 1 bis 4, worin der Träger einen langkettigen Kohlenwasserstoff darstellt.

6. Stoffzusammensetzung in Aerosolform nach einem der Ansprüche 1 bis 4, worin der Träger eine Silikonflüssigkeit darstellt.

7. Stoffzusammensetzung in Aerosolform nach einem der Ansprüche 1 bis 6, worin die quaternäre Ammoniumverbindung einen Methyl- oder Benzylrest und drei Alkylreste mit 14 bis 20 Kohlenstoffatomen enthält.

8. Stoffzusammensetzung in Aerosolform nach einem der Ansprüche 1 bis 7, worin der smektitische Ton Bentonit oder Hektorit darstellt.

9. Stoffzusammensetzung in Aerosolform nach einem der Ansprüche 1 bis 8, zusätzlich enthaltend einen niedrigmolekularen polaren organischen Aktivator für den smektitischen Ton.

10. Stoffzusammensetzung in Aerosolform nach Anspruch 9, worin der Aktivator Propylencarbonat oder einen Alkohol oder Keton mit niedrigem Molekulargewicht darstellt.

11. Stoffzusammensetzung in Aerosolform nach einem der Ansprüche 1 bis 10, worin das Treibmittel ein Isobutan enthaltendes Kohlenwasserstoffgemisch darstellt.

12. Verwendung der Stoffzusammensetzung in Aerosolform zum Blockieren von Allergenen nach einem der Ansprüche 1 bis 11, zur Herstellung eines Medikaments zum Schutz der Haut gegen Berührung mit einem Allergen.

13. Verwendung der Stoffzusammensetzung in Aerosolform zum Blockieren von Allergenen nach Teil A von Anspruch 1 und einem der Ansprüche 4 bis 10, für die Herstellung eines Medikaments zum Schutz der Haut gegen Berührung mit einem Allergen.

**14.** Verfahren zum Verhindern der Verunreinigung von Bekleidung und Gebrauchsgegenständen mit einem Allergen, wonach auf die Kleider und Gebrauchsgegenstände eine Blockierzusammensetzung aufgebracht wird, welche enthält:
1) 5 bis 15 Gew.% eines smektitischen Tons mit einer Kationenaustauschfähigkeit von mindestens 50 mVal je 100 g Ton, der mit einer quaternären Ammoniumverbindung mit mindestens einer Alkylgruppe, enthaltend mehr als 10 Kohlenstoffatome ionenausgetauscht worden ist, und
2) 95 to 85 Gew.% eines Trägers.

**Patentansprüche für folgende Vertragsstaaten : AT, ES, GR**

**1.** Verfahren zur Herstellung einer Stoffzusammensetzung in Aerosolform zum Blockieren von Allergenen, für die lokale Anwendung, enthaltend:
A. eine Blockier-Zusammensetzung, im wesentlichen bestehend aus
1) 5 bis 15 Gew.% eines smektitischen Tons mit einer Kationenaustauschfähigkeit von mindestens 50 mVal je 100 g Ton, der mit einer quaternären Ammoniumverbindung mit mindestens einer Alkylgruppe, enthaltend mehr als 10 Kohlenstoffatome ionenausgetauscht worden ist, und
2) 95 to 85 Gew.% eines Trägers; und
B. ein Aerosol-Treibmittel,
wobei nach diesem Verfahren die Komponente A1 mit der Komponente A2 vermischt und die Komponente B dem Gemisch zugesetzt wird.

**2.** Verfahren nach Anspruch 1, worin die Stoffzusammensetzung in Aerosolform 20 bis 35 Gewichtsteile Blockierzusammensetzung und 80 bis 65 Gewichtsteile Aerosol-Treibmittel enthält.

**3.** Verfahren nach Anspruch 1, worin die Stoffzusammensetzung in Aerosolform 30 Gewichtsteile Blockierzusammensetzung und 70 Gewichtsteile Aerosol-Treibmittel enthält.

**4.** Verfahren nach einem der Ansprüche 1 bis 3, worin der smektitische Ton einen hochaktivierten Ton darstellt.

**5.** Verfahren nach einem der Ansprüche 1 bis 4, worin der Träger einen langkettigen Kohlenwasserstoff darstellt.

**6.** Verfahren nach einem der Ansprüche 1 bis 4, worin der Träger eine Silikonflüssigkeit darstellt.

**7.** Verfahren nach einem der Ansprüche 1 bis 6, worin die quaternäre Ammoniumverbindung einen Methyl- oder Benzylrest und drei Alkylreste mit 14 bis 20 Kohlenstoffatomen enthält.

**8.** Verfahren nach einem der Ansprüche 1 bis 7, worin der smektitische Ton Bentonit oder Hektorit darstellt.

**9.** Verfahren nach einem der Ansprüche 1 bis 8, worin die Zusammensetzung zusätzlich einen niedrigmolekularen polaren organischen Aktivator für den smektitischen Ton enthält.

**10.** Verfahren nach Anspruch 9, worin der Aktivator Propylencarbonat oder einen Alkohol oder Keton mit niedrigem Molekulargewicht darstellt.

**11.** Verfahren nach einem der Ansprüche 1 bis 10, worin das Treibmittel ein Isobutan enthaltendes Kohlenwasserstoffgemisch darstellt.

**12.** Verwendung der nach einem der Ansprüche 1 bis 11 hergestellten Stoffzusammensetzung in Aerosolform zum Blockieren von Allergenen, zur Herstellung eines Medikaments zum Schutz der Haut gegen Berührung mit einem Allergen.

**13.** Verwendung der nach Teil A von Anspruch 1 und einem der Ansprüche 4 bis 10 hergestellten Stoffzusammensetzung in Aerosolform zum Blockieren von Allergenen, für die Herstellung eines Medikaments zum Schutz der Haut gegen Berührung mit einem Allergen.

**14.** Verfahren zum Verhindern der Verunreinigung von Bekleidung und Gebrauchsgegenständen mit einem Allergen, wonach auf die Kleider und Gebrauchsgegenstände eine Blockierzusammensetzung aufgebracht wird, welche enthält:

1) 5 bis 15 Gew.% eines smektitischen Tons mit einer Kationenaustauschfähigkeit von mindestens 50 mVal je 100 g Ton, der mit einer quaternären Ammoniumverbindung mit mindestens einer Alkylgruppe, enthaltend mehr als 10 Kohlenstoffatome ionenausgetauscht worden ist, und

2) 95 to 85 Gew.% eines Trägers.

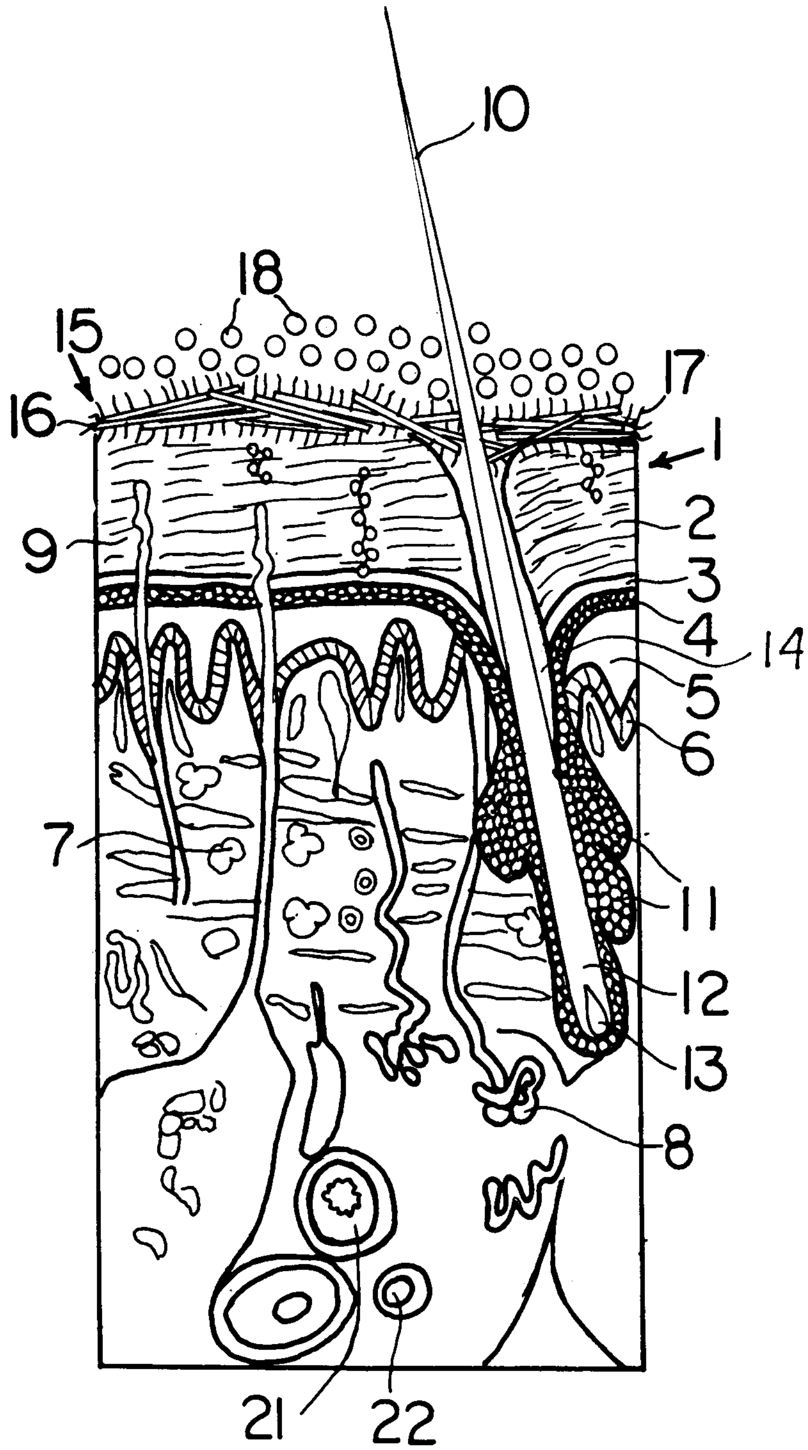


FIG 1

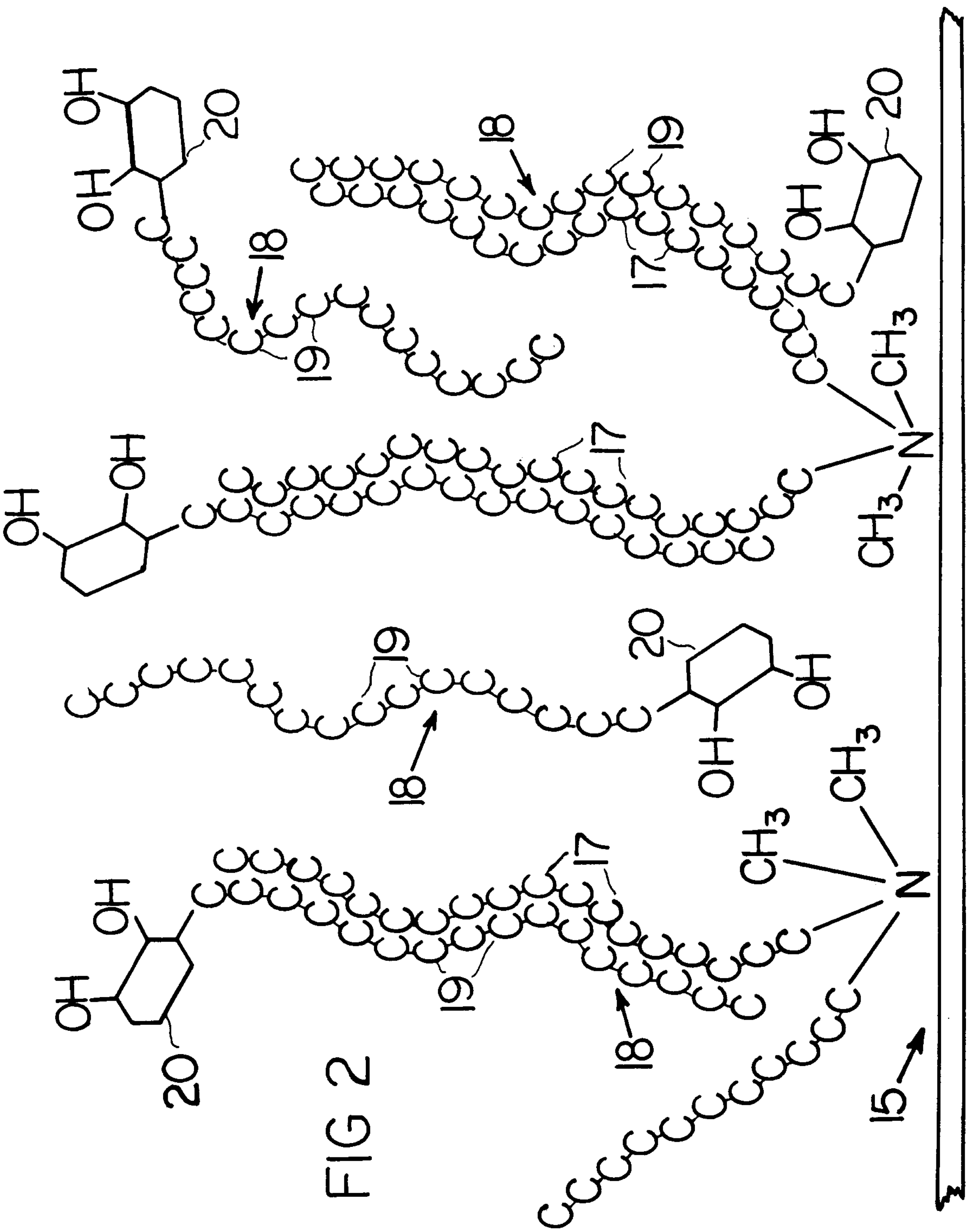

FIG 2